# EUROPEAN PATENT APPLICATION

(11) **EP 4 681 618 A1**
(43) Date of publication of application: **21.01.2026**
(21) Application number: 25189752.6
(22) Date of filing: 15.07.2025
(51) Int. Cl.: A61B 5/00, G16H 20/30

(54) **A FORCE APPLICATION APPARATUS**

(30) Priority: 16.07.2024 GB 202410351
(71) Applicant: Lilliput UK Ltd, West Midlands WV109RU (GB)
(72) Inventor: SHARIFI, Mohammed, deceased (GB)
(74) Representative: Swindell & Pearson Limited

(57) **Abstract**

A force application apparatus comprising receiving means configured to receive data comprising position and force data; force application means configured to apply a force to at least one part of a human body; and control means configured to control the force application means to apply force to at least one part of a human body based on the received position and force data.

## Description

### TECHNOLOGICAL FIELD

Examples of the disclosure relate to a force application apparatus, an apparatus, and a system.

### BACKGROUND

The development and advancement of wireless connectivity is improving the ability to control devices remotely. Doctors are increasingly utilizing remote access in order to provide healthcare to patients.

### BRIEF SUMMARY

According to various, but not necessarily all, examples there is provided a force application apparatus comprising: receiving means configured to receive data comprising position and force data; force application means configured to apply a force to at least one part of a human body; and control means configured to control the force application means to apply force to at least one part of a human body based on the received position and force data.

The force application apparatus may comprise positioning means configured to position the force application means to apply the force to the at least one part of a human body based on the received position data.

The positioning means may comprise a plurality of guides arranged to move the force application means.

The control means may be configured to control the positioning means.

The force application means may comprise at least one contact sensor configured to sense the force applied to the at least one part of a human body when the force application means applies a force to the at least one part of a human body.

The contact sensor may be arranged at an end of the force application means and the contact sensor is configured to at least partially contact the at least one part of a human body when the force application means applies a force to the at least one part of a human body.

The control means may be configured to receive sensed force data from the contact sensor and control the force application means based on the sensed force data.

The force application apparatus may comprise at least one scanning means configured to scan the at least one part of a human body.

The force application apparatus may comprise a transmitting means configured to transmit scanned data from the scanning means.

The force application apparatus may comprise at least one temperature sensor configured to sense the skin temperature of the at least one part of a human body.

The force application apparatus may comprise at least one light emitting means. The control means may be configured to control the force application means to apply the force based on an output from the light emitting means.

The force application apparatus may comprise a rotatable surface configured to receive a portion of a human body. The control means may be configured to adjust an angle of the rotatable surface based on the received data. The received data may comprise an indication of an angle for the rotatable surface.

The force application apparatus may comprise a frame. The frame may comprise at least one hand support. The at least one hand support may comprise at least one emergency control means configured to remove any force applied by the force application means.

The force application apparatus may comprise a display.

The display may be attached to the frame.

According to various, but not necessarily all, examples there is provided an apparatus comprising: a plurality of sensors, wherein each sensor is configured to sense an applied force; and transmitting means configured to transmit control data to a force application apparatus. The control data comprises position and force data based on a force applied to at least one of the plurality of sensors. The control data is configured to control the force application apparatus to apply a corresponding force to at least one part of a human body based on the position and force data.

The plurality of sensors may be arrangeable on a surface of an artificial human body part.

The plurality of sensors may be at least partially housed in an artificial human body part.

The plurality of sensors may be arranged to be removably positionable on an artificial human body part.

The apparatus may comprise a cover configured to house the plurality of sensors. The cover may be configured to be positionable on an artificial human body part.

The plurality of sensors may be located on a surface of an artificial body part. The artificial human body part may comprise a plurality of adjustable portions. The adjustable portions may be configured to adjust the shape and size of the artificial human body part.

The apparatus may comprise receiving means configured to receive scanned data of a human body part. The apparatus may be configured to adjust the adjustable portions based on the received scanned data.

The plurality of sensors may be positioned in a grid.

According to various, but not necessarily all, examples there is provided a system comprising the force application apparatus and the apparatus.

According to various, but not necessarily all, examples there is provided a system comprising: a scanning means configured to at least partially scan at least one part of a human body; a transmitting means configured to transmit scanned data from the scanning means; a receiving means configured to receive the scanned data from the transmitting means; and an artificial human body part comprising adjustable means. The adjustable means is configured to adjust the shape of the artificial human body part based on the received scanned data.

The description of a function and/or action should additionally be considered to also disclose any means suitable for performing that function and/or action. Functions and/or actions described herein can be performed in any suitable way using any suitable method.

According to various, but not necessarily all, embodiments there is provided examples as claimed in the appended claims.

While the above examples of the disclosure and optional features are described separately, it is to be understood that their provision in all possible combinations and permutations is contained within the disclosure. It is to be understood that various examples of the disclosure can comprise any or all the features described in respect of other examples of the disclosure, and vice versa. Also, it is to be appreciated that any one or more or all the features, in any combination, may be implemented by/comprised in/performable by an apparatus, a method, and/or computer program instructions as desired, and as appropriate. The description of a function should additionally be considered to also disclose any means suitable for performing that function.

### BRIEF DESCRIPTION

Some examples will now be described with reference to the accompanying drawings in which:
FIG. 1 shows an example of a system described herein;
FIGs. 2A-2C shows an example of an apparatus;
FIG. 3 shows a first example of a force application apparatus;
FIG. 4 shows a second example of a force application apparatus;
FIG. 5 shows an example of a positioning means and a force application means;
FIGs. 6A-6C illustrate movement of the example positioning means;
FIG. 7 shows a third example of the force application apparatus; and
FIGs. 8A-8C illustrate an example of a rotatable surface of the third example force application apparatus.

The figures are not necessarily to scale. Certain features and views of the figures can be shown schematically or exaggerated in scale in the interest of clarity and conciseness. For example, the dimensions of some elements in the figures can be exaggerated relative to other elements to aid explication. Similar reference numerals are used in the figures to designate similar features. For clarity, all reference numerals are not necessarily displayed in all figures.

### DETAILED DESCRIPTION

Embodiments of the present disclosure relate to a system comprising an apparatus and a force application apparatus.

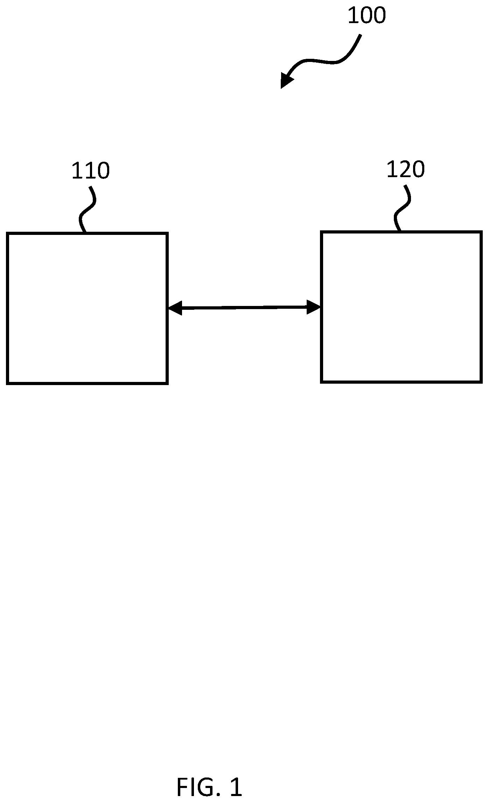

FIG. 1 illustrates a system 100 comprising an apparatus 110 and a force application apparatus 120. The apparatus 110 and the force application apparatus 120 are in communication (for example wireless communication) with each other. Data may be communicated between the apparatus 110 and the force application apparatus 120 via short range radio communications such as Wi-Fi or Bluetooth, for example, or over long-range cellular radio links. The apparatus 110 and the force application apparatus 120 may comprise a communications interface such as, for example, a radio transceiver for communication of data.

In some examples, the communication is a one-way communication, where data is sent from the apparatus 110 to the force application apparatus 120. In some examples, the communication is a two-way communication, where data is sent and received between the apparatus 110 and the force application apparatus 120. For example, the apparatus 110 may send data to the force application apparatus 120, and may receive data from the force application apparatus 120.

The force application apparatus 120 may be configured to be controlled remotely, as described further below, via the apparatus 110. For example, a user of the apparatus 110, such as a doctor, may control the force application apparatus 120 to assess a body part of a user of the force application apparatus 120, such as a patient.

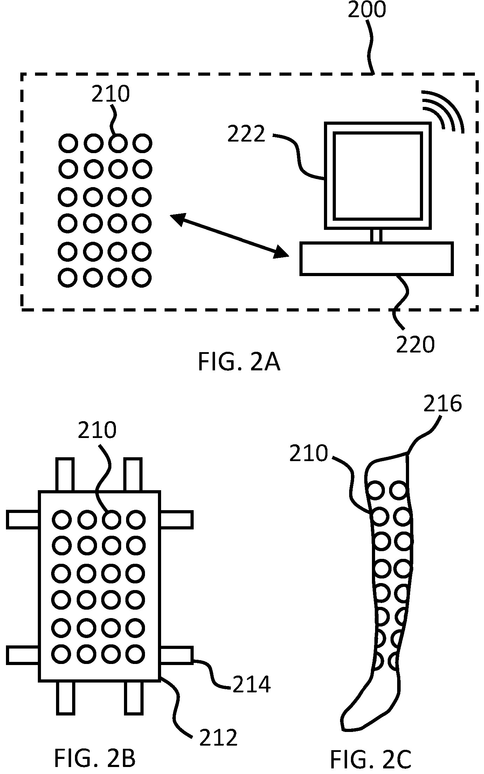

FIG. 2A illustrates an example of an apparatus 200, which may be the same as the apparatus 110 of FIG. 1. The apparatus 200 comprises a plurality of sensors 210 and transmitting means 220.

Each sensor of the plurality of sensors 210 is configured to sense an applied force. The plurality of sensors 210 may be a plurality of force-sensing sensors, for example force-sensing resistors. In particular, the plurality of sensors 210 are configured to sense a force applied by a human hand or fingers. The plurality of sensors 210 are configured to sense the extent of the applied force. In other words, each sensor of the plurality of sensors 210 is configured to sense how much force is applied, when a force is applied to that sensor.

In the illustrated example of FIG. 2A, the plurality of sensors 210 are positioned, or positionable, in a grid. However, it is envisaged that the plurality of sensors 210 may be positioned in any arrangement. For example, the distance between adjacent sensors (illustrated in a vertical and horizontal manner) may not be uniform for all sensors. Some of the sensors may be positioned, grouped, closer together. For example, this may be to account for the sensors representing a three-dimensional curved space, with a particular section of the space requiring a greater density of sensors. Therefore, it is envisaged that the number of sensors and the arrangement illustrated in FIGs. 2A-2C is merely an example, and more or fewer sensors may be used.

The plurality of sensors 210 are configured to communicate with the transmitting means 220. For example, the plurality of sensors 210 may be in wired or wireless communication with the transmitting means 220. In the illustrated example of FIG. 2A, the transmitting means 220 is a computer. The transmitting means 220 is configured to receive data from the plurality of sensors 210. The transmitting means 220 may be in direct communication with the plurality of sensors 210, as illustrated in FIG. 2A, or may be in indirect communication. When the transmitting means 220 is in indirect communication with the plurality of sensors 210, the apparatus 200 may comprise an additional component configured to receive data from the plurality of sensors 210, and configured to provide the received data (either processed or unprocessed) to the transmitting means 220 (either directly or indirectly). The received data from the plurality of sensors 210 comprises which sensor/sensors of the plurality of sensors 210 sensed an applied force, and how much force was sensed. The transmitting means 220 may be configured to receive data from the plurality of sensors 210 in real-time. In other words, the transmitting means 220 may be configured to receive data from the plurality of sensors 210 as the force is applied to at least one of the sensors. The transmitting means 220 may be configured to determine the greatest, in other words largest, force applied to each sensor. An additional component of the apparatus 200 may be configured to determine the greatest, in other words largest, force applied to each sensor, and may be configured to provide the determined values (either processed or unprocessed) to the transmitting means 220 (either directly or indirectly).

The transmitting means 220 of the apparatus 200 is configured to transmit control data to a force application apparatus, such as the force application apparatus 120 of FIG. 1. The transmitting means 220 can comprise any suitable means/structure(s)/arrangement(s)/component(s) configured to transmit. In examples, the transmitting means 220 can be considered at least one transmitter, at least one transceiver, at least one transmitting arrangement, at least one transmitting structure, at least one transmitting component, transmitting circuitry and so on.

The control data comprises position and force data based on a force applied to at least one of the plurality of sensors 210. The control data is configured to control the force application apparatus 120 to apply a corresponding force to at least one part of a human body based on the position and force data. The position data is based on which of the sensor/sensors of the plurality of sensors 210 the force was applied to. Each sensor may be mapped to a particular position for the force application apparatus 120. For example, each sensor of the plurality of sensors 210 may be mapped to particular position of a human body part, and the force application apparatus 120 is configured to apply a corresponding force to that position. The force data is based on the extent of the force sensed by the at least one of the plurality of sensors 210, for example the greatest force sensed by the at least one of the plurality of sensors 210.

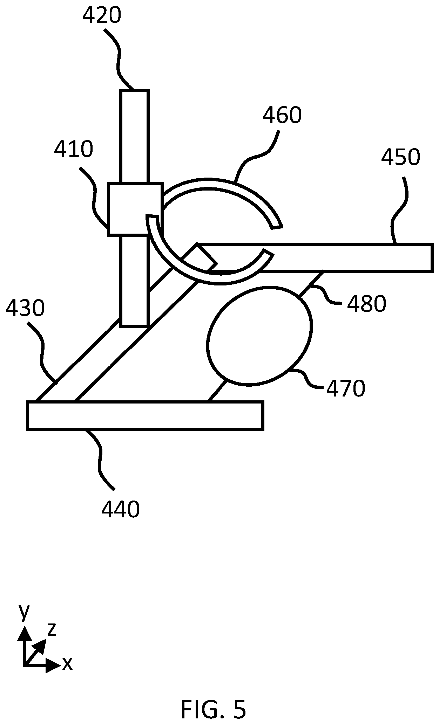

The transmitting means 220 may be configured to transmit data indicating an angle for a rotatable surface, such as a rotatable surface of the force application apparatus 120 (see below with reference to FIGs. 5, 7 and 8A-8C), to the force application apparatus 120. The data indicating an angle may be selected by a user of the apparatus 200. The data indicating an angle may be included in the control data transmitted by the transmitting means 220. The rotatable surface is configured to contract and/or relax certain muscles based on the angle of the rotatable surface. The rotatable surface is configured to receive at least a part of a user's body (such as a foot to be positioned on the rotatable surface), and the angle of the rotatable surface is configured to contract and/or relax certain muscles of the user's body.

In some examples, the apparatus 200 comprises receiving means. The receiving means can comprise any suitable means/structure(s)/arrangement(s)/component(s) configured to receive. In examples, the receiving means can be considered at least one receiver, at least one receiving arrangement, at least one receiving structure, at least one receiving component, receiving circuitry and so on.

The illustrated computer in FIG. 2A may comprise receiving means. The receiving means may be configured to receive data from the force application apparatus 120, such as scanned data of a human body part. This may be applicable, for example, when a two-way communication is used between the apparatus 200 and the force application apparatus 120. Scanned data may be from a camera, a three-dimensional scanner, or an x-ray machine.

The apparatus 200 may comprise a cover 212 configured to house the plurality of sensors 210. An example of a cover 212 is illustrated in FIG. 2B. The cover 212 may, for example, be a wrap, a sleeve, a sock, a pad, or the like. In the illustrated example of FIG. 2B, the cover 212 comprises a pad (or the like) to house the plurality of sensors 210. The plurality of sensors 210 may be positioned in a grid, as illustrated, or another arrangement as discussed above. The cover 212 may comprise at least one fastener 214 configured to affix the cover 212. In the illustrated example, the fasteners 214 are straps, or the like. For example, the straps may be in the form of a belt, or may comprise corresponding hook and loop portions, or buttons with corresponding buttonholes. The fastener(s) may take a different form, such as an adhesive surface.

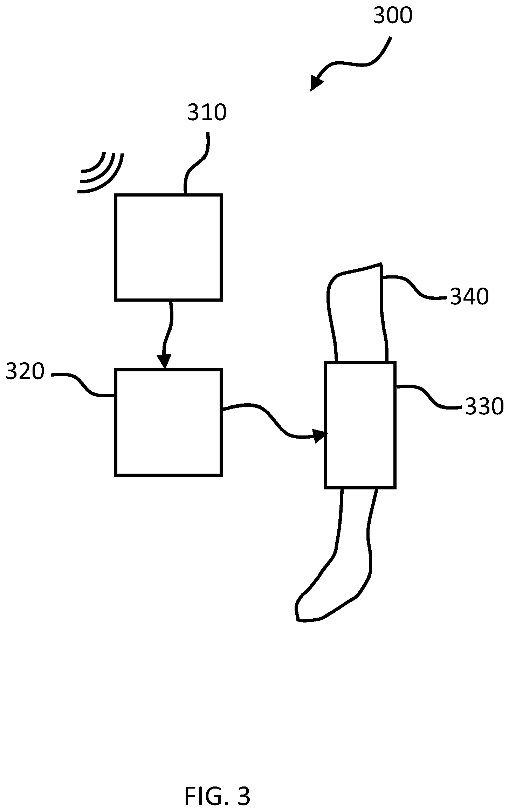

The apparatus 200 may comprise, see for example FIG. 2C, an artificial human body part 216. The plurality of sensors 210 may be arranged to be removably positionable on the artificial human body part 216. The plurality of sensors 210 may be arrangeable on a surface of the artificial human body part 216. In some examples, the cover 212 is configured to be affixed to the artificial human body part 216. The cover 212 may therefore be configured to be positionable on the artificial human body part 216. When the cover 212 is affixed to the artificial human body part 216 (FIG. 2C), each sensor of the plurality of sensors 210 is positioned on (or proximal to) the surface of the artificial human body part 216. Each sensor may therefore be mapped to a particular position for the force application apparatus 120, where the artificial human body part 216 corresponds to a human body part for the force application apparatus 120 (as described below with reference to FIGs. 3-7). For example, the position of each sensor may correspond to a similar position of a human body part (same body part as the artificial human body part 216), and the force application apparatus 120 is configured to apply a force to that position.

In some examples, the plurality of sensors 210 is at least partially housed in the artificial human body part 216. FIG. 2C may therefore illustrate the cover 212 affixed to the artificial human body part 216, or illustrate the plurality of sensors 210 at least partially housed in the artificial human body part 216. In both instances, the plurality of sensors 210 are positioned around the artificial human body part 216. The plurality of sensors 210 can therefore sense an applied force at particular positions around the artificial human body part 216.

In some examples, the artificial human body part 216 comprises a plurality of adjustable portions. The adjustable portions may be configured to adjust the shape and size of the artificial human body part 216. The apparatus 200 may be configured to adjust the adjustable portions based on received scanned data. The received scanned data can therefore be used to adjust the shape and size of the artificial human body part 216 to more accurately resemble the human body part which was scanned. In other words, the artificial human body part 216 may replicate the shape, size, contours etc. of the scanned human body part. This advantageously results in the plurality of sensors 210, located on a surface of the artificial body part 216, being more accurately positioned in relation to the scanned human body part. In the illustrated example of FIG. 2A, the computer is configured to adjust the adjustable portions of the artificial body part 216 based on the received scanned data.

The adjustable portions may comprise motorised compartments which are configured to be moved independently based on received scanned data. The adjustable portions may comprise inflatable pockets which are configured to be independently inflated/deflated based on received scanned data.

The artificial human body part 216 may be configured to alter the colour of particular portions of the artificial human body part 216. For example, the artificial human body part 216 may comprise a plurality of coloured lights. In some examples, the apparatus 200 is configured to alter the colour of particular portions of the artificial human body part 216. Received scanned data of the human body part may comprise data relating to colouration of the scanned human body part, and the artificial human body part 216, or the apparatus 200, may be configured to adjust at least one of the plurality of coloured lights in accordance with the colouration data. In other examples, the artificial human body part 216 may comprise at least one flexible display configured to display at least a portion of the received scanned data, for example where colouration has been determined. Colouration may represent any determined bruises, swelling, or other abnormalities from the received scanned data. The apparatus 200 (for example the illustrated computer) may be configured to analyse the received scanned data to determine such colouration. For example, an image analysis may be performed on the received scanned data. The received scanned data may include an image analysis with any determined colouration.

In FIG. 2C, the artificial human body part 216 is illustrated as an artificial leg. It is also envisaged that another part of the human body could be used, for example an artificial limb, such as an artificial arm, or a torso.

The illustrated apparatus 200 comprises a display 222. The apparatus 200 may be configured to display received scanned data on the display 222. For example, the received scanned data may be one or more pictures, a video, or one or more X-ray images. In some examples, the apparatus 200 is configured to display a video conversation on the display 222. For example, the user of the apparatus 200 may be able to see and speak to the user of the force application apparatus 120.

FIG. 3 illustrates an example of the force application apparatus 300, which may be the same as the force application apparatus 120 of FIG. 1. The force application apparatus 300 comprises receiving means 310, control means 320, and force application means 330.

The receiving means 310 is configured to receive data comprising position and force data. For example, the receiving means 310 is configured to receive data from the apparatus 200, for example from transmitting means 220 of the apparatus 200. The receiving means 310 may be configured to receive data indicating an angle for a rotatable surface, such as the rotatable surface described below with reference to FIGs. 5, 7 and 8A-8C.

The receiving means 310 can comprise any suitable
means/structure(s)/arrangement(s)/component(s) configured to receive, for example, at least one signal comprising information. In examples, the receiving means 310 can be considered at least one receiver, at least one receiving arrangement, at least one receiving structure, at least one receiving component, receiving circuitry and so on.

The control means 320 is configured to control the force application means 330 to apply force to at least one part of a human body 340 based on the received position and force data. In some examples, the control means 320 is configured to control the position of the force application means 330. For example, the control means 320 may be configured to control one or more motors used to position the force application means 330 based on the received position data.

The control means 320 can comprise any suitable
means/structure(s)/arrangement(s)/component(s) configured to control, for example, the force application means. In examples, the control means 320 can be considered at least one controller, at least one controlling arrangement, at least one controlling structure, at least one controlling component, controlling circuitry and so on.

In some examples, the control means 320 is a controller. Implementation of a controller may be as controller circuitry. The controller may be implemented in hardware alone, have certain aspects in software including firmware alone or can be a combination of hardware and software (including firmware). The control means 320 may be implemented using instructions that enable hardware functionality, for example, by using executable instructions in a general-purpose or special-purpose processor that may be stored on a machine readable storage medium (disk, memory etc.) to be executed by such a processor.

The force application means 330 is configured to apply a force to at least one part of a human body 340. The at least one part of a human body 340 is the same body part as the artificial human body part 216 for the apparatus 200. In the illustrated example of FIG. 3, the human body part 340 is a portion of a leg. However, it is envisaged that the force application means 330 may apply a force to another human body part, such as a limb (for example the whole leg, or an arm) or a torso. It is envisaged that the force application means 330 may be configured to apply a force to at least one part of a non-human body, for example an animal body. In such examples, reference herein to a part of a human body, a human body part and an artificial human body part may instead relate to any particular animal body.

The force application means 330 can comprise any suitable
means/structure(s)/arrangement(s)/component(s) configured to apply a force, for example, to at least one part of a human body. In examples, the force application means 330 can be considered at least one force applicator, at least one force application arrangement, at least one force application structure, at least one force application component, force application circuitry and so on.

The force application means 330 may comprise at least one mechanical arm. The mechanical arm is configured to apply a force to the human body part 340 based on the received data. In particular, the force application apparatus 300, specifically the control means 320, may be configured to position the at least one mechanical arm based on the position data, and apply a force based on the force data. Each mechanical arm may comprise at least one finger. Each finger may be configured to be independently moveable. In other words, each finger may be configured to be individually controlled based on the received data, for example controlled by the control means 320. A contact sensor and/or a temperature sensor may be positioned at the end of each mechanical arm, or at the end of one or more fingers. In other words, the contact sensor and/or temperature sensor may contact the human body part 340 when the force application means 330 applies a force to the human body part 340.

The force application means 330 may comprise one or more actuators, each arranged to apply a force to a part of a human body 340. The one or more actuators may be moveable in relation to the human body part 340, based on the received data. The force application apparatus 300, specifically the control means 320, may be configured to position the one or more actuators based on the position data, and apply a force substantially based on the force data. A contact sensor and/or a temperature sensor may be positioned at the end of each actuator. In other words, the contact sensor and/or temperature sensor may contact the human body part 340 when the force application means 330 applies a force to the human body part 340.

The force application means 330 may comprise one or more (sealed or sealable) pockets to receive a fluid, such as air or a liquid. The force application means 330 may be configured to at least partially fill each pocket with fluid. This may, for example, be controlled by the control means 320. The amount of fluid received in a pocket may be based on the received data. The greater the force to be applied to the human body part 340 by the pocket, the greater the amount of fluid received by that pocket. The force application apparatus 300 may be configured to fill a pocket with fluid based on the position data, and fill the pocket with fluid to apply a force substantially based on the force data. The force application apparatus 300 may be configured to remove fluid from the pockets, thus removing any force applied by the pockets. A contact sensor and/or a temperature sensor may be positioned on a surface of one or more pockets. In particular, on a surface of the pocket which contacts the human body part 340 when a force is applied.

The force application means 330 may comprise a cover (such as a wrap, a sleeve, a sock, a pad, or the like). The cover may comprise a plurality of force applicators, such as actuators. Each force applicator may be mapped to a sensor of the plurality of sensors 210 of the apparatus 200. Therefore, the force applied to the human body part 340 by the plurality of force applicators may be based on the received data, and therefore based on which sensor of the plurality of sensors 210 sensed a force. The plurality of force applicators may be configured to apply a force based on the received force data. A contact sensor and/or a temperature sensor may be positioned at the end of one or more force applicators. In other words, the contact sensor and/or temperature sensor may contact the human body part 340 when the force application means 330 applies a force to the human body part 340. In some examples, the cover comprises one or more pockets to receive a fluid, as described above.

The force application means 330 may comprise at least one contact sensor configured to sense the force applied to the at least one part of a human body 340 when the force application means 330 applies a force to the at least one part of a human body 340. The contact sensor may be a force-sensing sensor, for example force-sensing resistor, or a point sensor. The contact sensor may be arranged at an end of at least part of the force application means 330. The contact sensor is configured to at least partially contact the at least one part of a human body 340 when the force application means 330 applies a force to the at least one part of a human body 340. The contact sensor is to be positioned on the surface of the force application means 330 which contacts the at least one part of a human body 340 when the force application means 330 applies the force to the at least one part of a human body 340. The control means 320 may be configured to receive sensed force data from the contact sensor and control the force application means 330 based on the sensed force data. In other words, the control means 320 may use the sensed force data from the contact sensor to determine any difference between the intended force and the applied force. The control means 320 may be configured to control the force application means 330 in real-time, using sensed force data as a feedback loop.

The contact sensor may be configured to sense distance to the human body part 340. The control means 320 may be configured to receive sensed distance data from the contact sensor to assist in positioning the force application means 330 and to control the force application means 330 to account for any movement to make initial contact with a surface of the human body part 340.

In some examples, the force application apparatus 300 comprises at least one scanning means configured to scan the at least one part of a human body 340. The scanning means can comprise any suitable
means/structure(s)/arrangement(s)/component(s) configured to scan, for example, the at least one part of a human body. In examples, the scanning means can be considered at least one scanner, at least one camera, at least one three-dimensional scanner, at least one scanning arrangement, at least one scanning structure, at least one scanning component, scanning circuitry and so on.

In some examples, the at least one scanning means is configured to be positioned on positioning means, or the force application means 330. For example, the control means 320 may be configured to control positioning means to scan the at least one part of a human body 340 using the at least one scanning means. The at least one scanning means may be an apparatus configured to detect electromagnetic radiation, such as at least one of a (optical) camera, a three-dimensional scanner (which may be configured to detect optical wavelengths), or a portable x-ray machine.

The force application apparatus 300 may be configured to determine areas of colouration from scanned data of the scanning means.

The control means 320 may be configured to adjust the received data based on scanned data from the at least one scanning means. For example, the received position data may be adjusted based on the scanned data. The scanned data provides more accurate details regarding the human body part 340 the force is to be applied to. The control means 320 may be configured to control the force application means 330 based on the scanned data.

The force application apparatus 300 may comprise at least one temperature sensor configured to sense the skin temperature of the at least one part of a human body 340. The temperature sensor may be an infrared sensor. In some examples, the temperature sensor is a part of the contact sensor.

The force application apparatus 300 may be configured to process the temperature data from the at least one temperature sensor. For example, the force application apparatus 300 may be configured to determine whether a temperature of the temperature data is above an upper threshold. If the sensed temperature in the temperature data is determined to be above the upper threshold, the force application apparatus 300 may be configured to scan the human body part using the scanning means. For example, if the sensed temperature of the human body part is too high, then the force application apparatus 300 may then acquire additional information, such as a photo, a video, a scan or an x-ray. This may be advantageous as the force application apparatus 300 is configured to pre-emptively obtain additional information for determined high temperatures. In a similar manner, the force application apparatus 300 may be configured to determine whether a temperature of the temperature data is below a lower threshold. If the sensed temperature in the temperature data is determined to be below the lower threshold, the force application apparatus 300 may be configured to scan the human body part using the scanning means. For example, if the sensed temperature of the human body part is too low, then the force application apparatus 300 may then acquire additional information, such as a photo, a video, a scan or an x-ray.

The force application apparatus 300 may comprise at least one light emitting means. The control means 320 may be configured to control the force application means 330 to apply the force based on an output from the light emitting means. In other words, the at least one light emitting means may be used to assist with correct positioning of the force application means 330. The at least one light emitting means may comprise a laser. The control means 320 may be configured to use the at least one light emitting means to determine distance between the force application means 330 and the human body part 340. This advantageously provides the control means 320 with additional information for controlling the force application means 330 to apply the correct force based on the determined distance. For example, the control means 320 may be configured to position the force application means 330 based on the determined distance such that the force applied to the human body part 340 is accurate. In some examples, the at least one light emitting means comprises at least one light emitting diode.

The light emitting means can comprise any suitable
means/structure(s)/arrangement(s)/component(s) configured to emit light. In examples, the light emitting means can be considered at least one light emitter, at least one light, at least one light emitting arrangement, at least one light emitting structure, at least one light emitting component, light emitting circuitry and so on.

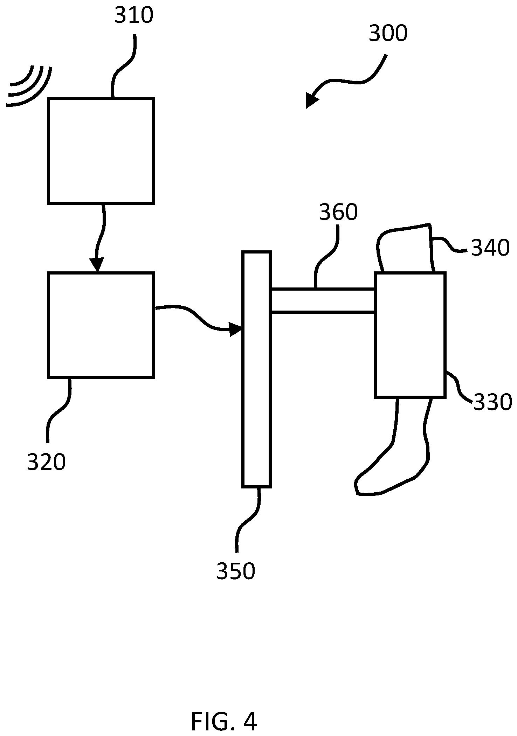

The force application apparatus 300 may comprise positioning means 350 configured to position the force application means 330 to apply the force to the at least one part of a human body 340 based on the received position data. An example of the positioning means 350 is illustrated in FIG. 4. In the illustrated example, the control means 320 is configured to control the positioning means 350. This may be, for example, based on the received data.

The positioning means 350 can comprise any suitable
means/structure(s)/arrangement(s)/component(s) configured to position the force application means 330. In examples, the positioning means 350 can be considered at least one positioning arrangement, at least one positioning structure, at least one positioning component, positioning circuitry and so on.

The force application means 330 is connected to the positioning means 350 by attachment means 360. In some examples, the positioning means 350 comprises the attachment means 360. It may be that the force application means 330 comprises the attachment means 360.

The attachment means 360 can comprise any suitable
means/structure(s)/arrangement(s)/component(s) configured to attach the force application means 330 to the positioning means 350. In examples, the attachment means 360 can be considered at least one attachment, at least one attachment arrangement, at least one attachment structure, at least one attachment component, attachment circuitry and so on.

In some examples, the force application apparatus 300 is configured to receive data to control the positioning means 350 remotely. A user (for example a doctor) of apparatus 200 may remotely control the positioning means 350 of the force application apparatus 300, for example in real-time.

A scanning means, as described above, may be positioned on the positioning means 350. The scanning means may be positioned on a further positioning means configured to position the scanning means to scan the at least one part of a human body 340. The further positioning means may be moved independently from the positioning means 350. A user (for example a doctor) of apparatus 200 may remotely control the further positioning means of the force application apparatus 300, for example in real-time.

The further positioning means can comprise any suitable
means/structure(s)/arrangement(s)/component(s) configured to position the scanning means. In examples, the further positioning means can be considered at least one positioning arrangement, at least one positioning structure, at least one positioning component, positioning circuitry and so on.

The force application apparatus 300 may comprise transmitting means configured to transmit data, for example to the apparatus 200. For example, the force application apparatus 300 may be configured to transmit at least one of the following: scanned data from scanning means; temperature data from at least one temperature sensor; contact data from at least one contact sensor; video footage from a camera; audio data from a microphone.

The transmitting means can comprise any suitable
means/structure(s)/arrangement(s)/component(s) configured to transmit, for example, at least one signal comprising information. In examples, the transmitting means can be considered at least one transmitter, at least one transceiver, at least one transmitting arrangement, at least one transmitting structure, at least one transmitting component, transmitting circuitry and so on.

FIG. 5 illustrates an example of force application means and positioning means of the force application apparatus 300. The force application means may be as described above with reference to FIGs. 3 and 4, and the positioning means may be as described above with reference to FIG. 4.

The positioning means comprises attachment means 410 (which may be as described above) and a plurality of guides 420-450. In the illustrated example, the force application means comprises two mechanical arms 460, each arm configured to apply a force based on the received data. Each mechanical arm of the force application means is individually controllable, for example by the control means 320. In such examples, a control sensor (as described above) and/or a temperature sensor (as described above) may be positioned on at least one of the mechanical arms 460. A scanning means (as described above) may be positioned along at least one of the mechanical arms 460. The mechanical arms 460 of the force application means of FIG. 5 may be replaced with another type of force application means, such as actuators, pockets, cover, etc.

The mechanical arms 460 of the force application means are connected, or connectable, to the attachment means 410. In other examples, the force application means may comprise the attachment means 410, and the force application means is attached to the positioning means by the attachment means 410. The attachment means 410 is configured to attach the mechanical arms 460 to a guide 420 of the positioning means. The attachment means 410 may also be configured to open/close the mechanical arms 460 with respect to each other. The control means may be configured to control the attachment means 410 to move the mechanical arms 460.

The positioning means is configured to position the force application means in three different (perpendicular) directions. In the illustrated example, the guides 420-450 of the positioning means are configured to position the force application means in the x-, y-, and z-directions. The positioning means may comprise a respective set of motors to position the force application means for a given axis. This advantageously provides good accuracy when applying a force to the human body by the force application means. In particular, the mechanical arms of the force application means can be positioned accurately to ensure force is applied within a few millimetres of the received position data.

One or more of the guides 420-450 may comprise a linear slide. Each linear slide may comprise a rail and at least one carriage, where the carriage is slidably moveable along the rail. Each guide 420-450 may comprise at least one motor configured to position the force application means. For example, each motor may be configured to slidably move a respective carriage along a respective rail based on the received position data. In other words, each motor may be configured to slidably move at least one carriage along the rail of the respective guide, therefore moving the force application means 330 based on the received position data in a given direction. The guides 420-450 may comprise a threaded portion and the at least one carriage is at least partially attached to the threaded portion. Each motor may be configured to slidably move a respective carriage by rotating the threaded portion which causes the carriage to move. Each motor may be configured to operate to move the force application means 330 in both directions of a given axis. For example, a single motor may be used to move the force application means 330 in a positive z-direction and in a negative z-direction, using guide 430 of FIG. 5.

In the illustrated example, the positioning means 350 comprises four guides 420-450. One guide 420 is aligned with the y-axis, one guide 430 is aligned with the z-axis, and two guides 440 and 450 are aligned with the x-axis. The positioning means 350 may comprise more or fewer guides.

The force application apparatus may also comprise a rotatable surface 470, an example is provided in FIG. 5. The rotatable surface 470 is configured to receive a portion of a human body, for example a foot or a hand. The angle of the rotatable surface 470 is configured to be adjustable. For example, an indication of an angle for the rotatable surface 470 may be comprised in the received data or received separately, for example transmitted by the apparatus 110 and 200. The rotatable surface 470 may be a rotatable plate, or a rotatable ring.

In the illustrated example, the rotatable surface 470 is configured to rotate around the z-axis. For example, one or more bars 480 may be used to control rotation of the rotatable surface 470. In some examples, the bars 480 are connected to the rotatable surface 470 on an underside of the rotatable surface 470 (i.e., the surface of the rotatable surface 470 which faces the negative y-direction of FIG. 5). A single bar 480 may extend from one guide to another guide, connected to the rotatable surface 470 on an underside of the rotatable surface 470. The rotatable surface 470 has the advantage of allowing contraction and/or relaxation of muscles of the human body when the force application means 460 applies a force.

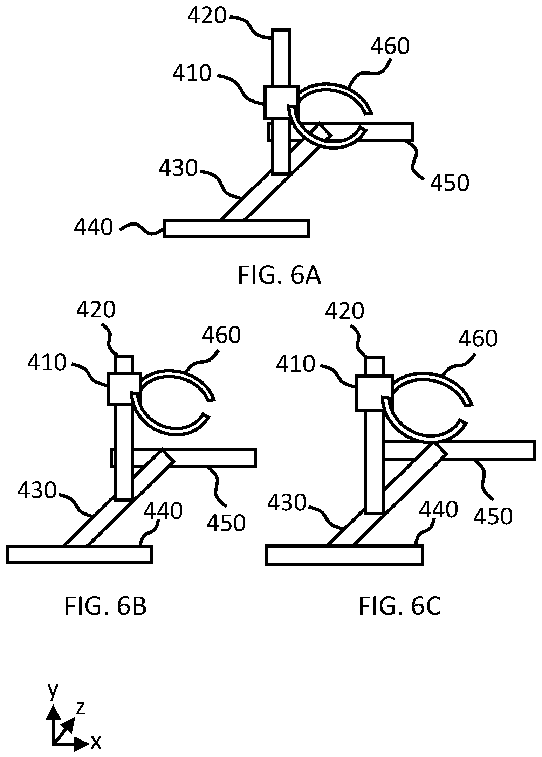

Examples of the movement of the force application means 330 by the positioning means 350 is illustrated in FIGs. 6A-6C. FIG. 6A illustrates the force application means 330 having been moved in the positive x-direction in relation to the position illustrated in FIG. 5, along guides 440 and 450. FIG. 6B illustrates a further movement of the force application means 330 in the positive y-direction, along guide 420. Finally, FIG. 6C illustrates movement of the force application means 330 in the negative z-direction, in relation to the position illustrated in FIG. 6B, along guide 430.

The positioning means 350, or the attachment means 410, may be configured to extend the mechanical arms in the positive x-direction. For example, the mechanical arms may be attached to a guide 420 of the positioning means 350 which can be moveable in the x-direction, other than the use of the guides 440 and 450 as described above.

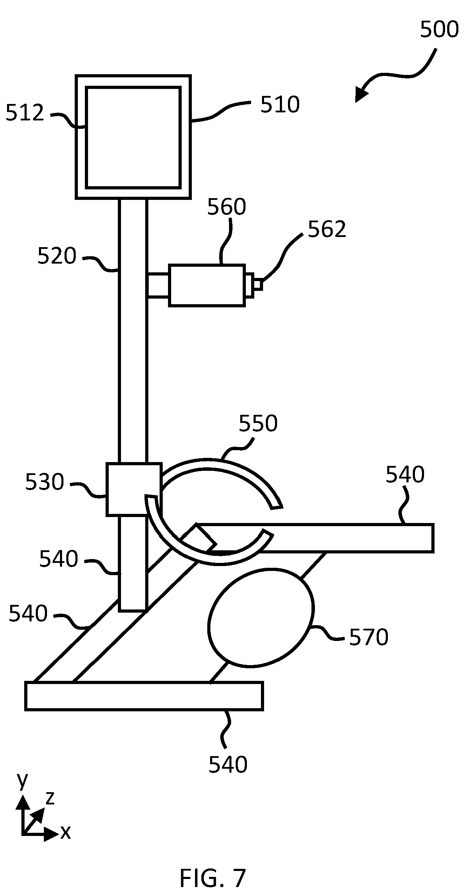

FIG. 7 illustrates an example of a force application apparatus 500. The force application apparatus 500 of FIG. 7 performs in a similar manner as described above, for example the force application apparatus 300 with reference to FIGs. 3 to 6.

The illustrated force application apparatus 500 comprises receiving means and control means in a computer 510. In other words, a computer 510 may comprise receiving means and control means. The computer 510 may comprise transmitting means, as described above. In some examples, the receiving means and the transmitting means comprises at least one transceiver.

In FIG. 7, the computer 510 is connected to a display 512. In some examples, the computer 510 comprises the display 512. The force application apparatus 500, or the computer 510, may comprise a webcam (not shown). The force application apparatus 500 may be configured to display various information on the display 512. For example, the force application apparatus 500 may be configured to display at least one of a video, instructions, upcoming actions, or results on the display 512.

The force application apparatus 500 may be configured to establish a real-time conversation. In other words, the user of the apparatus 200 may be able to communicate, in real-time, with the user of the force application apparatus 500. In some examples, the communication is a video conversation.

The force application apparatus 500 of FIG. 7 comprises a frame 520. The computer 510 and/or the display 512 may be attached, or affixed, to the frame 520, as illustrated. Attachment means 530 may be attached to at least a portion of the frame 520. The attachment means 530 may be the same as the attachment means 410 described above with reference to FIGs. 5 and 6. The frame 520 may comprise at least a portion of the positioning means, such as a guide 540 of the positioning means. The illustrated guides 540 may be similar to the guides 420-450 of positioning means 350 described above in relation to FIGs. 3-6C. The plurality of guides 540 are therefore configured to position force application means 550, which is the same as the force application means 330 of FIGs. 5-6C.

In addition, the illustrated frame 520 comprises a hand support 560. In particular, the illustrated force application apparatus 500 is arranged for applying a force to a human leg. The hand support 560 therefore provides support for the user of the force application apparatus 500.

The hand support 560 may comprise at least one emergency control means 562, such as an emergency button, configured to remove any force applied by the force application means 550, as illustrated in FIG. 7. In other words, the at least one emergency control means 562 is configured to override control means (for example control means 320) of the force application apparatus 500. In some examples, the at least one emergency control means 562 is configured to return a rotatable surface (for example rotatable surface 570) to a horizontal angle (see below).

The force application apparatus 500 of FIG. 7 also comprises a rotatable surface 570, similar to the rotatable surface 470 described above with reference to FIG. 5. The rotatable surface 570 is configured to receive a portion of a human body, for example a foot or a hand. Control means of the force application apparatus 500 is configured to adjust an angle of the rotatable surface 570, which may be based on the received data. In other words, the received data may comprise an indication of an angle for the rotatable surface 570.

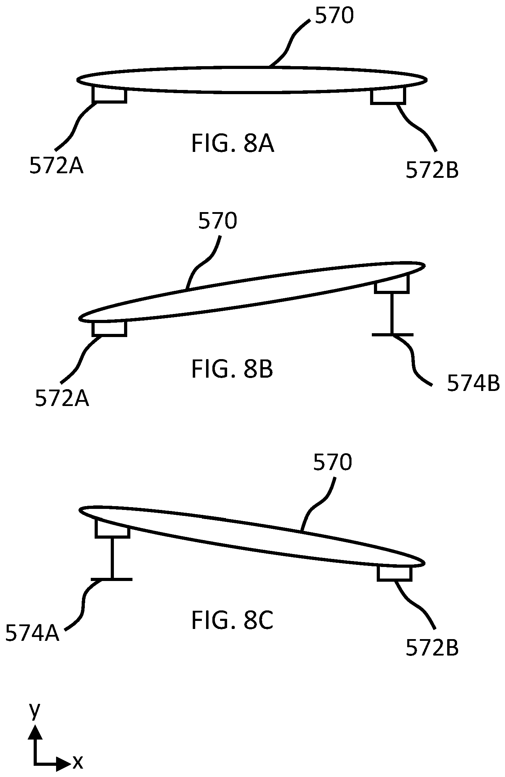

An example of different angles for the rotatable surface 570 is illustrated in FIGs. 8A-8C. For example, the force application apparatus 500 may comprise a plurality of motors 572A and 572B under the rotatable surface 570. Each motor 572A and 572B may be configured to extend, and contract, a respective extendable leg 574A and 574B. When the extendable legs 574A and 574B are not extended, the rotatable surface 570 is substantially flat, as illustrated in FIG. 8A. In other words, the rotatable surface 570 is substantially parallel to the x-axis. Each extendable leg 574A and 574B may be equally extended to contact a surface, for example a floor, to provide additional support to the rotatable surface 570, and to prevent rotation of the rotatable surface 570 while keeping the rotatable surface 570 substantially flat.

As one of the extendable legs 574A or 574B extends, it causes the rotatable surface 570 to tilt to an angle. In particular, the extendable leg 574A or 574B may contact a surface (such as a floor), and extension of the extendable leg 574A or 574B after contact with the surface causes the rotatably surface 570 to tilt. The desired angle of the rotatable surface 570 can be achieved by extending the required extendable leg 574A or 574B to a certain length. FIG. 8B illustrates the rotatable surface 570 having an angle such that the rotatable surface 570 is inclined in the positive x-direction (by using the motor 572B to extend the extendable leg 574B). Similarly, FIG. 8C illustrates the rotatable surface 570 having an angle such that the rotatable surface 570 is inclined in the negative x-direction (by using the motor 572A to extend the extendable leg 574A).

When a user of the force application apparatus 500 places a body part, such as a foot, on the rotatable surface 570, different muscles can be contracted and/or relaxed depending on whether the rotatable surface 570 is flat, or inclined in the positive x-direction (as per FIG. 8B) or in the negative x-direction (as per FIG. 8C). This can be advantageous as certain muscles can be contracted and/or relaxed when the force application means applies a force.

Where a structural feature has been described, it may be replaced by means for performing one or more of the functions of the structural feature whether that function or those functions are explicitly or implicitly described.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to 'comprising only one...' or by using 'consisting.'

In this description, the wording 'connect', 'couple' and 'communication' and their derivatives mean operationally connected/coupled/in communication. It should be appreciated that any number or combination of intervening components can exist (including no intervening components), i.e., to provide direct or indirect connection/coupling/communication. Any such intervening components can include hardware and/or software components.

As used herein, the term "determine/determining" (and grammatical variants thereof) can include, not least: calculating, computing, processing, deriving, measuring, investigating, identifying, looking up (for example, looking up in a table, a database, or another data structure), ascertaining and the like. Also, "determining" can include receiving (for example, receiving information), accessing (for example, accessing data in a memory), obtaining and the like. Also, " determine/determining" can include resolving, selecting, choosing, establishing, and the like.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can', or 'may' refers to a particular instance in a class of examples. A property of the instance can be a property of only that instance or a property of the class or a property of a sub-class of the class that includes some but not all the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

The description of a feature, such as an apparatus or a component of an apparatus, configured to perform a function, or for performing a function, should additionally be considered to also disclose a method of performing that function. For example, description of an apparatus configured to perform one or more actions, or for performing one or more actions, should additionally be considered to disclose a method of performing those one or more actions with or without the apparatus.

Although features have been described with reference to certain examples, those features may also be present in other examples whether described or not.

The term 'a', 'an' or 'the' is used in this document with an inclusive not an exclusive meaning. That is any reference to X comprising a/an/the Y indicates that X may comprise only one Y or may comprise more than one Y unless the context clearly indicates the contrary. If it is intended to use 'a', 'an' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

The above description describes some examples of the present disclosure however those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description of the examples of the present disclosure.

Whilst endeavouring in the foregoing specification to draw attention to those features believed to be of importance the Applicant may seek protection via the claims in respect of any patentable feature or combination of features hereinbefore referred to and/or shown in the drawings whether or not emphasis has been placed thereon.

## Claims

1. A force application apparatus comprising:
receiving means configured to receive data comprising position and force data;
force application means configured to apply a force to at least one part of a human body; and
control means configured to control the force application means to apply force to at least one part of a human body based on the received position and force data.

2. The force application apparatus of claim 1, wherein the force application apparatus comprises positioning means configured to position the force application means to apply the force to the at least one part of a human body based on the received position data, wherein, optionally, the positioning means comprises a plurality of guides arranged to move the force application means, wherein, optionally, the control means is configured to control the positioning means.

3. The force application apparatus of any preceding claim, wherein the force application means comprises at least one contact sensor configured to sense the force applied to the at least one part of a human body when the force application means applies a force to the at least one part of a human body, wherein, optionally, the contact sensor is arranged at an end of the force application means and the contact sensor is configured to at least partially contact the at least one part of a human body when the force application means applies a force to the at least one part of a human body, wherein, optionally, the control means is configured to receive sensed force data from the contact sensor and control the force application means based on the sensed force data.

4. The force application apparatus of any preceding claim, wherein the force application apparatus comprises at least one scanning means configured to scan the at least one part of a human body, wherein, optionally, the force application apparatus comprises a transmitting means configured to transmit scanned data from the scanning means.

5. The force application apparatus of any preceding claim, wherein the force application apparatus comprises at least one temperature sensor configured to sense the skin temperature of the at least one part of a human body.

6. The force application apparatus of any preceding claim, wherein the force application apparatus comprises at least one light emitting means, and wherein the control means is configured to control the force application means to apply the force based on an output from the light emitting means.

7. The force application apparatus of any preceding claim, wherein the force application apparatus comprises a rotatable surface configured to receive a portion of a human body, wherein the control means is configured to adjust an angle of the rotatable surface based on the received data.

8. The force application apparatus of any preceding claim, wherein the force application apparatus comprises a frame, wherein the frame comprises at least one hand support, and wherein the at least one hand support comprises at least one emergency control means configured to remove any force applied by the force application means.

9. The force application apparatus of any preceding claim, wherein the force application apparatus comprises a display.

10. An apparatus comprising:
a plurality of sensors, wherein each sensor is configured to sense an applied force; and
transmitting means configured to transmit control data to a force application apparatus, wherein the control data comprises position and force data based on a force applied to at least one of the plurality of sensors, and wherein the control data is configured to control the force application apparatus to apply a corresponding force to at least one part of a human body based on the position and force data.

11. The apparatus of claim 10, wherein the plurality of sensors are arrangeable on a surface of an artificial human body part, wherein, optionally, the plurality of sensors are at least partially housed in an artificial human body part.

12. The apparatus of claim 11, wherein the plurality of sensors are arranged to be removably positionable on an artificial human body part, wherein, optionally, the apparatus comprises a cover configured to house the plurality of sensors, and wherein the cover is configured to be positionable on an artificial human body part.

13. The apparatus of any one of claims 11 or 12, wherein the plurality of sensors are located on a surface of an artificial body part, wherein the artificial human body part comprises a plurality of adjustable portions, wherein the adjustable portions are configured to adjust the shape and size of the artificial human body part, wherein, optionally, the apparatus comprises receiving means configured to receive scanned data of a human body part, and wherein the apparatus is configured to adjust the adjustable portions based on the received scanned data.

14. A system comprising:
the force application apparatus of any one of claims 1 to 9 and the apparatus of any one of claims 10 to 13.

15. A system comprising:
a scanning means configured to at least partially scan at least one part of a human body;
a transmitting means configured to transmit scanned data from the scanning means;
a receiving means configured to receive the scanned data from the transmitting means; and
an artificial human body part comprising adjustable means, wherein the adjustable means is configured to adjust the shape of the artificial human body part based on the received scanned data.
